(19) Europäisches Patentamt / European Patent Office / Office européen des brevets

(11) **EP 3 426 313 B1**

(12) **EUROPEAN PATENT SPECIFICATION**

(45) Date of publication and mention of the grant of the patent:
**07.07.2021 Bulletin 2021/27**

(21) Application number: **17715286.5**

(22) Date of filing: **28.02.2017**

(51) Int Cl.:
*A61L 29/16* (2006.01)       *A01N 31/02* (2006.01)
*A01N 37/44* (2006.01)

(86) International application number:
**PCT/IN2017/000052**

(87) International publication number:
**WO 2017/154015 (14.09.2017 Gazette 2017/37)**

(54) **BROAD SPECTRUM ANTIMICROBIAL&ANTICOAGULANT COMPOSITION**

ANTIMIKROBIELLE UND GERINNUNGSHEMMENDE ZUSAMMENSETZUNG MIT BREITEM SPEKTRUM

COMPOSITION ANTIMICROBIENNE ET ANTICOAGULANTE À LARGE SPECTRE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **07.03.2016 IN 201621007809**

(43) Date of publication of application:
**16.01.2019 Bulletin 2019/03**

(73) Proprietor: **AGGARWAL, Dinesh**
**Raipur, C.G. 492099 (IN)**

(72) Inventor: **AGGARWAL, Dinesh**
**Raipur, C.G. 492099 (IN)**

(74) Representative: **CSY London**
**10 Fetter Lane**
**London EC4A 1BR (GB)**

(56) References cited:
**WO-A1-2015/048559      US-A1- 2009 170 947**

- **T.-C. Chou: "Theoretical Basis, Experimental Design, and Computerized Simulation of Synergism and Antagonism in Drug Combination Studies", Pharmacological Reviews, vol. 58, no. 3, 1 September 2006 (2006-09-01), pages 621-681, XP055151376, ISSN: 0031-6997, DOI: 10.1124/pr.58.3.10**

## Description

**FIELD OF INVENTION:**

The present invention relates to a composition, a synergistic combination that provides effective antimicrobial and anticoagulant effects. In an embodiment, the composition is part of a catheter lock-flush solution providing optimum antimicrobial and anticoagulant effects.

**BACKGROUND OF THE INVENTION:**

Infections are a significant problem in many fields where sanitary conditions are important, such as in healthcare. Problematic infections may arise from bacterial, fungal, amoebic, protozoan and/or viral organisms. Challenges are encountered both in preventing infection, and in reducing or eliminating the infection once it is established. Infected environments may include but not limited to surfaces of objects, fluids and fluid conduits and/or humans or animals.

One problematic source of infections in the medical and veterinary fields is found in catheters, particularly in in-dwelling catheters. Catheters have become essential in the management of critical care patients, yet the inside of a catheter is often the major source of infection. Catheters are used for delivery of fluids, blood products, drugs, nutrients, hemo-dialysis, hemo-filtration, peritoneal dialysis, retrieval of blood samples, monitoring of patient conditions, and the like. Trans-cutaneous catheters often become infected through skin penetration of the catheter. It has been found that seventy percent (70%) of all nosocomial bloodstream infections occur in patients with central venous catheters. Daouicher et al. 340, 1-8, New England Journal Of Medicine (1999).

In particular, during some procedures, a catheter must be implanted, and remain implanted in a patient for a relatively long period of time, e.g. over thirty days. Intravenous (IV) therapy catheters and urinary catheters typically remain implanted for a substantial period of time. As a result of trauma to the areas of insertion, and pain to the patients, such catheters can't be removed and implanted frequently. Catheter-borne bacteria are implicated as a primary

source of urinary tract infections. Patients who receive a peripherally inserted central catheter during pregnancy have also been found to be at significant risk for infectious complications. "Complications Associated With Peripherally Inserted Central Catheter Use During Pregnancy" AM. J. OBSTET. GYCOL. 188(5):1223-5 May 2003. In addition, central venous catheter infection, resulting in catheter related sepsis, has been cited as the most frequent complication during home parenteral nutrition. Clinical Nutrition, 21(1):33-38, 2002. Because of the risk of infections, catheterization may be limited to incidences when the procedure is absolutely necessary. This seriously compromises patient health.

In customary medical practice, after a medical procedure involving the use of catheter, the catheter is flushed with saline and then filled with a liquid, such as saline or a heparin solution, to prevent blood from clotting inside of the catheter, to inhibit the patient's blood from backing up into the catheter, and to prevent gases from entering the catheter. The liquid that is used to flush the catheter is referred to as a "lock-flush," and the liquid used to fill the catheter following flushing or during periods of non-use is referred to as a "lock" solution.

To reduce problems associated with clotting and thrombus formation, it is now common to "lock" intravascular access catheters between successive uses. Locking typically involves first flushing the catheter with saline to remove blood and other substances from the catheter lumen. After the catheter has been flushed, an anti-coagulant solution, typically heparin, is then injected to displace the saline and fill the lumen. The heparin-locking solution prevents blood from entering the lumen and actively inhibits clotting and thrombus formation within the lumen. While some thrombus may still form at the distal tip of the catheter, the formation is usually minimal and presents few problems. It has further been proposed to combine various antimicrobial substances with the locking solution in order to inhibit infection at the same time that thrombus formation is being inhibited.

Traditionally, catheters have been locked with normal saline or heparin solutions. Heparin and saline are sometimes used in combination. Normal saline is generally used to lock short-term peripheral intravenous catheters, but saline has no anticoagulant or antimicrobial activity. Heparin solutions are generally used to lock vascular catheters. Heparin has anticoagulant activity but it does not function as an antimicrobial and does not prevent or ameliorate infections. There are also strong indications that heparin in lock solutions may contribute to heparin-induced thrombocytopenia, a serious bleeding complication that occurs in a subset of patients receiving heparin injections.

An emerging trend and recommendation from the Center for Infectious Disease (CID), USA is to treat existing catheter infections systemically with either a specific or a broad range antibiotic. Use of an antibiotic in a lock solution to prevent infection is not recommended. The use of antibiotics to treat existing catheter infections has certain risks, including: (1) the risk of antibiotic-resistant strains developing; (2) the inability of the antibiotic to kill sessile, or deep-layer bio-film bacteria, which may require the use of antibiotics at toxic concentrations; and (3) the high cost of prolonged antibiotic therapy. Catheters coated with a disinfectant or antibiotic materials are available. These coated catheters may only provide limited protection for a relatively short period of time.

In general, free-floating organisms may be vulnerable to antibiotics. However, bacteria and fungi may become impervious to antibiotics by attaching to surfaces and producing a slimy protective substance, often referred to as extra-cellular polymeric substance (EPS), polysaccharide covering or glycocalyx. As the microbes proliferate, more than 50 genetic up or down regulations may occur, resulting in the formation of a more antibiotic resistant microbial bio-film. One article attributes two-thirds of the bacterial infections that physicians encounter to bio-films. Science News, 1-5 Jul. 14, 2001.

The antibiotic resistance of bio-films, coupled with complications of antibiotic use, such as the risk of developing antibiotic resistant strains, has made antibiotic treatment an unattractive option. As a result, antibiotic use is limited to symptomatic infections and prophylactic antibiotics are not typically applied to prevent contamination. Because the bio-film can act as a selective phenotypic resistance barrier to most antibiotics, the catheter must often be removed in order to eradicate a catheter related infection. Removal and replacement of the catheter is time consuming, stressful to the patient, and complicates the medical procedure. Therefore, there are attempts to provide convenient and effective methods for killing organisms, and especially those dwelling inside of catheters, without the necessity of removing the catheter from the body. Antimicrobial lock/flush solutions are needed to salvage indwelling catheters in patients requiring continuous intravenous therapy.

According to Justo JA1, Bookstaver PB (Infect Drug Resist. 2014 Dec 12;7:343-63) Antibiotic lock therapy (ALT) for the prevention and treatment of catheter-related bloodstream infections is a simple strategy in theory, yet its real-world application may be delayed or avoided due to technical questions and/or logistical challenges. Justo et al. analyzed various aspects of ALT, including preparation information for a variety of antibiotic lock solutions (i.e., aminoglycosides, beta-lactams, fluoroquinolones, folate antagonists, glycopeptides, glycylcyclines, lipopeptides, oxazolidinones, polymyxins, and tetracyclines) and common clinical issues surrounding ALT administration. Additionally, Justo et al. summarized detailed data regarding concentrations, additives, stability/compatibility, and dwell times. Finally, Justo et al. also described logistical challenges such as lock preparation procedures, use of additives (e.g., heparin, citrate, or ethylene diamine tetra acetic acid), timing of initiation and therapy duration, optimal dwell time and catheter accessibility, and risks of ALT.

Catheter locking solutions comprising taurolidine, citric acid and sodium citrate have been proposed. A publication (Kidney International, September 2002) describes the use of a 70% alcohol solution as a lock solution for a subcutaneous catheter port.

According to Pierce DA, Rocco MV (Pharmacotherapy 2010 Nov;30(11):1150-8) trisodium citrate (also known as sodium citrate or citrate) is an efficacious alternative to heparin as a locking solution. Citrate exerts both its anticoagulant and antimicrobial properties by chelating calcium to disrupt the normal coagulation pathway and by interfering with the formation of bio-film and the bacterial cell wall. Pierce et al. speculate that, citrate is at least equivalent to heparin as an anticoagulant and antimicrobial agent for catheter locking, and in some clinical studies citrate was shown to be superior. However, higher concentration of sodium citrate (i.e. 46.7%) used in the study, raised safety concerns. The systemic hypo-calcemic effects that were reported with citrate 46.7% have not been observed with citrate 4% in clinical trials, and the risk of systemic anticoagulation and bleeding was shown to be lower than that with unfractionated heparin. In addition, most comparative cost data indicate that citrate is a more cost-effective alternative than heparin.

John E. Moran, Stephen R. Ash (Locking Solutions for Hemodialysis Catheters; Heparin and Citrate-A Position Paper by ASDIN, Semin Dial. 2008 Sep-Oct;21(5):490-2) found that the following solutions are suitable choices for lock solution to maintain patency of tunneled central venous catheters for dialysis: Heparin 1,000 U/ml and 4% Sodium citrate. Risks from systemic anticoagulation are lower with heparin 1,000 U/ml and 4% sodium citrate, compared with higher concentrations of heparin (5,000 and 10,000 U/ml). The need for use of tPA for maintaining catheter patency is increased by using heparin lock at 1,000 U/ml, vs. higher concentrations.

U.S. Pat. No. 6,350,251 disclose internal prosthetic devices such as catheters or ports including a bio-cidal lock comprising an anticoagulant and a non-antibiotic biocide.

EP 1644024 A2 patent maintains that alcohol based compositions are unable to maintain effective compositional consistency. Alcohol is volatile. Consequently, the composition gets diluted over time. Accordingly, the alcohol-based compositions do not provide long-lasting antimicrobial activity. This results in the need for repeated flushing of the catheter and renewal of the antimicrobial composition when the time between uses of the catheter is long.

Hence, there is a need for improved methods and compositions to prevent and destroy infections in a variety of environments. Such compositions should have a broad range of antimicrobial properties, yet safe. In particular, the compositions should be capable of penetrating bio-films to eradicate the organisms comprising the bio-films. The methods and compositions should be safe enough to be used as a preventive measure as well as in the treatment of existing infections.

Ethylene diamine tetra acetic acid, and salts thereof, (EDTA) has been used for systemic detoxification treatment and as an anticoagulant in blood samples for some time. Thus its use for medical treatment and applications is established. The use of disodium EDTA and calcium disodium EDTA in combination with other compounds to enhance antimicrobial properties of these other compounds has been studied and practiced. It has been discovered that many stand-alone salts of Ethylene diamine tetra acetic acid (EDTA) are effective antimicrobial agents and that specific salts are more effective than others. In particular, it has been discovered that certain salts of EDTA exhibit antimicrobial (both antifungal and antibacterial) properties superior to those of the disodium salt in common use. In particular, di-potassium and ammonium EDTA are superior to di-sodium EDTA, and tetra-sodium EDTA (TEDTA) has been found to be preferred over disodium, ammonium, and di-potassium.

Mannitol is a white crystalline solid and taste like sucrose. It is classified as sugar alcohol. Medically it is utilized to reduce acutely raised intracranial pressure. It may also be used for certain cases of kidney failure with low urine output, decreasing pressure in the eye, to increase the elimination of certain toxins, and to treat fluid build-up. Further, it has been reported that Mannitol not only helps in improving lung function by facilitating mucus clearance in Cystic Fibrosis, but also affects antibiotic sensitivity in bio-films. It does so via an active, physiological response (Barraud N, Buson A, Jarolimek W, Rice SA (2013) Mannitol Enhances Antibiotic Sensitivity of Persister Bacteria in Pseudomonas aeruginosa Bio-films. PLoS ONE 8(12): e84220. doi:10.1371/journal.pone.0084220).

Ethanol is used in medical wipes and in common antibacterial hand sanitizer gels, often at a concentration of about 62% (percentage by volume, not weight) as an antiseptic. Ethanol kills organisms by denaturing their proteins and dissolving their lipids. Ethanol is effective against most bacteria and fungi, and many viruses, but is ineffective against bacterial spores. In addition, Ethanol has also been used to reduce catheter occlusion caused by lipid material.

However a recent clinical trial on 50% ethanol lock solution where locking was performed from 1-3 hours showed no reduction in infection relative to heparin locking (Cmich C. J., Duster M., Jones A., and Maki D. G. - Prospective Randomized Double-Blind Trial of an Ethanol Lock for Prevention of CLABSI Proceedings 49" Interscience Conference on Antimicrobial Agents and Chemotherapy, San Francisco, CA Sept. 12-16, 2009).

Ethanol with multiple ingredients has been tried. For example, i.e. in combination with EDTA along with other antimicrobial substances including antibiotics have been evaluated as a catheter lock solutions.

Issam Raad, et al (Antimicrob. Agents Chemother. January 2007 vol. 51 no. 1 78-83) determined the activity of minocycline, EDTA, and 25% ethanol, alone or in combination. It was evaluated against - methicillin-resistant S. *aureus* and C. *parapsilosis* catheter-related bloodstream infection strains in two established models of Bio-film colonization. M-EDTA in 25% ethanol was significantly more effective in rapidly eradicating the growth or re-growth of the stated microorganisms when compared with other solutions-minocycline, EDTA, M-EDTA, 25% ethanol, and EDTA in ethanol. They concluded that M-EDTA in 25% ethanol is highly effective at rapidly eradicating *S. aureus* and *C. parapsilosis* embedded in bio-film adhering to catheter segments.

Beatriz Passerini de Rossi et al (Journal of Medical Microbiology (2012), 61, 1248-1253) compared the in-vitro activity of ethanol, EDTA and levofloxacin (Levo), alone or in combination. The activity was evaluated on bio-films of *Stenotrophomonas maltophilia* recovered from patients with catheter-related bloodstream infections (CRBSIs) at a university hospital in Argentina. The biomass reduction estimated via crystal violet staining was primarily dependent on the isolate. The most effective agents were 25 and 40% ethanol. Further, the results suggested that ethanol (at lower concentrations) alone or in combination with chelator when utilized for short periods, can decontaminate the line from *S. maltophilia* in cases of CRBSI. In addition, ethanol in conjunction with systemic antibiotics can potentially help the retention of precious vascular catheters.

Patent no. EP 1644024 A2 describes antimicrobial solutions that comprise at least one alcohol, at least one antimicrobial agent and at least one chelator and/or anticoagulant. The patent also provided methods for rapidly reducing a microbe or a virus from surfaces including surfaces of indwelling medical devices and organic surfaces such as skin and sutures, and inorganic surfaces such as hospital equipment, pipelines etc.

Patent No. EP1628655 A4 describes antiseptic compositions comprising at least one salt of EDTA. These compositions have broad-spectrum antimicrobial and antifungal activity. In addition, they have anticoagulant properties. Further, the antiseptic compositions have demonstrated activity in penetrating and breaking down microbial slime, or bio-films. They are considered safe for human and medical uses. They may be used as prophylactic preparations to prevent infection, or to reduce the proliferation of and/or eliminate existing or established infections.

WO2015048559 A1 discloses a catheter locking formulation and method to prepare same; and US 200901 70947A1 discloses disinfectant compositions comprising PHMB and EDTA salt.

Thus, there still remains a need for a composition that is safe for lock flush solution preferably with safe and well tested non-antibiotic components like EDTA and ethanol which can provide maximum synergistic activity that can eradicate bacteria/fungi as well as other microorganisms and preformed bio-films rapidly. In addition, certain components may be added which not only helps in increasing bio-film susceptibility to antimicrobial activity but also helps in controlling characteristics such as viscosity, density, osmolality for optimum action.

**OBJECTS AND SUMMARY:**

The main objective of the invention is to provide an effective antimicrobial composition.

Another objective of the present invention is to provide an "antibiotic and preservative free" catheter lock solution with optimum/enhanced antimicrobial activity.

Yet another objective of the present invention is to provide an ideal catheter lock flush solution having both antimicrobial and anticoagulant activity.

Another objective of the present invention is to provide a simple and cost effective composition having properties such as viscosity, density, osmolality similar to that of blood.

The invention describes a unique antimicrobial and anticoagulant composition as well as a method of preparation thereof. According to the invention, it is provided a cost effective catheter lock solution as defined in claim 1.

## BRIEF DESCRIPTION OF THE TABLES:

Table 1 shows the results of experiments of Minimum Inhibitory Concentration (MIC) Evaluation via Macro-dilution Method of Sodium Salt(s) of EDTA and Ethanol for S. aureus. From the data it is evident that the MIC values for Sodium Salt(s) of EDTA and Ethanol are 0.24 wt% & 25 vol% respectively.

Table 2 shows the results of experiments of MIC Evaluation via Macro-dilution Method of Sodium Salt(s) of EDTA and Ethanol for C. albicans. From the data it is evident that the MIC values for Sodium Salt(s) of EDTA and Ethanol are 0.24 wt% & 3.125 vol% respectively.

Table 3 shows the results of experiments of MIC Evaluation via Macro-dilution Method of Sodium Salt(s) of EDTA and Ethanol for P. aeruginosa. From the data it is evident that the MIC values for Sodium Salt(s) of EDTA and Ethanol are 0.24 wt% & 6.25 vol% respectively.

Table 4 shows the results of experiments of Checkerboard Titration using macro-dilution assay with S. aureus. The data suggests that the Fractional Inhibitory Concentration (FIC) index = 0.5 (synergy) for the Sodium Salt(s) of EDTA + Ethanol Combination.

Table 5 shows the results of experiments of Checkerboard Titration using macro-dilution assay with C. albicans. The data suggests that the FIC index = 0.5 (synergy) for the Sodium Salt(s) of EDTA + Ethanol Combination.

Table 6 shows the results of experiments of Checkerboard Titration using macro-dilution assay with P. aeruginosa. The data suggests that the FIC index = 0.98 (partial synergy) for the Sodium Salt(s) of EDTA + Ethanol Combination.

Table 7 shows the results of experiments of Rate Kill Assay. The data clearly suggests the synergistic action against S. aureus by Sodium Salt(s) of EDTA + Ethanol combination with a >2 log reduction at 1 hour and 3 hours when compared with the most active agent in the combination i.e. Ethanol alone.

Table 8 shows the results of experiments of Rate Kill Assay. The data clearly suggests the synergistic action against C. albicans by Sodium Salt(s) of EDTA + Ethanol combination with a log reduction of >2 at 1 hour when compared with the most active agent in the combination i.e. Ethanol alone.

Table 9 shows the results of experiments of Rate Kill Assay. The data clearly suggests the synergistic action against P. aeruginosa by Sodium Salt(s) of EDTA + Ethanol combination with a >2 log reduction at 1 and 3 hour when compared with the most active agent in the combination i.e. Ethanol alone.

Table 10 shows the results of experiments of Rate Kill Assay. The data clearly suggests the antimicrobial activity of Mannitol against S. aureus. In addition, it is evident that with increase in pH there is an enhancement in antimicrobial activity for combination formulations.

Table 11 shows the results of experiments of Rate Kill Assay. The data clearly suggests the antimicrobial activity of Mannitol against C. albicans. In addition, it is evident that with increase in pH the antimicrobial activity for combination formulations remains un-affected.

Table 12 shows the results of experiments of Rate Kill Assay. The data clearly suggests the antimicrobial activity of Mannitol against P. aeruginosa. In addition, it is evident that with increase in pH the antimicrobial activity for combination formulations remains un-affected.

Table 13 shows the results of experiments of Prothrombin Time (PT) Assay. This is primarily done to assess the anticoagulant efficacy of a formulation when compared with the standard controls. The data is presented in terms of International Normalized Ratio (INR). The data clearly suggests the Sodium Salt(s) of EDTA + Ethanol combinations have a higher PT when compared with the 10 IU/ml of heparin, which is considered a benchmark.

Table 14 shows the results of experiments of Prothrombin Time (PT) Assay. The data clearly suggests a) higher the EDTA concentration in combination, higher is the PT, b) with increase in pH there is a slight reduction in the PT and c) D-Mannitol doesn't affect have any negative effect on the PT for combination formulations.

**DETAILED DESCRIPTION OF THE INVENTION:**

In the following discussion, the terms "microbe" or "microbial" will be used to refer to microscopic organisms or matter, including fungal and bacterial organisms, and possibly including viral organisms, capable of infecting humans. The term "anti-microbial" will thus be used herein to refer to a material or agent that kills or otherwise inhibits the growth of fungal and/or bacterial and possibly viral organisms.

The term "disinfect" will be used to refer to the reduction, inhibition, or elimination of infectious microbes from a defined system. The term "disinfectant" will be used herein to refer to a one or more anti-microbial substances used either alone or in combination with other materials such as carriers, solvents, or the like.

The term "bactericidal activity" is used to refer to an activity that at least essentially kills an entire population of bacteria, instead of simply just reducing or inhibiting their growth. The term "fungicidal activity" is used to refer to an activity that at least essentially kills an entire population of yeast, instead of simply just reducing or inhibiting their growth. Contamination of conduits, e.g., catheters, poses serious and substantial health risks and bactericidal disinfection is a significant priority.

The term "infected system" will be used herein to refer to a defined or discrete system or environment in which one or more infectious microbes are or are likely to be present. Examples of infected systems include a physical space such as a bathroom facility or operating room, a physical object such as food or surgical tool, a biological system such as the human body, or a combination of a physical object and a biological system such as a catheter or the like arranged at least partly within a human body. Tubes and other conduits for the delivery of fluids, in industrial and healthcare settings, may also define an infected system. The term "anticoagulation" will be used to refer to the delay or abnormal blood clotting time when compared with a citrate control in a defined system. The term "anticoagulant" will be used herein refer to one or more anti-coagulant substances used either alone or in combination with other materials such as carriers, solvents, or the like.

The sodium salt(s) of EDTA, Ethanol and D-Mannitol may be used in compositions with distilled water or phosphate buffer saline as the solvent, or a combination thereof.

EDTA is used at low concentrations in many compositions, in combination with other active components, as a stabilizer or preservative agent. The compositions of the present disclosure comprise generally higher concentrations of EDTA.

The British Pharmacopoeia (BP) specifies that a 5% solution of di-sodium EDTA has a pH of 4.0 to 5.5. The BP also specifies a pH range of 7.0 to 8.0 for solutions of tri-sodium EDTA. At physiological pH, the sodium salts of EDTA exist as a combination of di-sodium and tri-sodium EDTA, with the tri-sodium salt(s) of EDTA being predominant. In the U.S., pharmaceutical "di-sodium" EDTA prepared for injection has generally been titrated with sodium hydroxide to a pH of 6.5 to 7.5. At this pH, the EDTA solution actually comprises primarily trisodium EDTA, with a lesser proportion of the di-sodium salt. Other compositions comprising sodium salts of EDTA that are used in medical or healthcare applications are generally adjusted to a pH that is substantially physiological.

Compositions comprising EDTA have a well-established safety profile in connection with medical usage and administration to humans. Doses of up to 3000 mg EDTA disodium are infused over 3 hours, on a daily basis, for the treatment of hyper-calcemia in humans. This dose is well tolerated. EDTA salts are also present, in combination with other components, in many solutions used in medical and human health applications, and have been established as safe for human use, both in vitro and in vivo. EDTA salts are readily available at a reasonable cost and are stable over time in solution.

Ethanol has a molecular Formula of $C_2H_5OH$ and a Molecular Weight of 46.07. Ethanol is also known as ethyl alcohol, grain alcohol, pure alcohol, hydroxyethane, drinking alcohol, ethyl hydrate and EtOH. Ethanol is readily available at a reasonable cost and is stable over time in solution.

Mannitol has a molecular formula $C_6H_{14}O_6$ and a molecular weight of 182.17. Mannitol is also known as mannite or manna sugar. It is readily available at a reasonable cost and is stable over time in solution.

A sodium salt(s) of EDTA has been shown to have some anti-coagulant effect. Ethanol has been shown to have some anti-coagulant effect. Anticoagulant activity of Mannitol is unknown. The combination of sodium salt(s) of EDTA, Ethanol and Mannitol has an anti-coagulant effect.

Embodiments of the disclosed composition comprise at least 0.06% of a sodium salt(s) of EDTA, by weight per volume solution (w/v) and up to 8% (w/v). Embodiments comprising up to 6 % (w/v) are preferred for many applications, compositions comprising at least 1% (w/v) of a sodium salt(s) of EDTA and up to 5% (w/v) are also preferred for certain applications and compositions comprising about 4% (w/v) of a sodium salt(s) of EDTA are especially preferred.

Embodiments of the disclosed composition comprise at least 6.25% Ethanol, by volume per volume solution (v/v) and up to 25% (v/v) Ethanol.

Embodiments comprising at least 10% (v/v) Ethanol are preferred for certain applications.

Embodiments of the disclosed composition comprise at least 0.5% of D-Mannitol, by weight per volume solution (w/v) and up to 10% (w/v). Embodiments comprising D-Mannitol up to 5% (w/v) are preferred for many applications, compositions comprising at least 0.5% (w/v) of D-Mannitol and up to 1% (w/v) are also preferred for certain applications and compositions comprising about 0.73% (w/v) of D-Mannitol are especially preferred.

The compositions comprising desired sodium salt(s) of EDTA, Ethanol and D-Mannitol concentrations for various applications may depend on the type of infection being treated and, to some degree, on the solvent used for disinfectant compositions. "Effective" concentrations of sodium salt(s) of EDTA, Ethanol and D-Mannitol in disinfectant compositions of the present disclosure for inhibitory, bactericidal, fungicidal, bio-film eradication and other purposes may be determined by routine experimentation.

In certain embodiments, disinfectant compositions of the present disclosure are at a pH range 7.5+/-0.5.

In some embodiments, the aqueous solvent is water or saline. Disinfectant compositions of the present disclosure consisting essentially of sodium salt(s) of EDTA, Ethanol and D-Mannitol are substantially free from other active substances having substantial antimicrobial and/or anti-fungal activity.

Substantial antimicrobial and/or anti-fungal activity, in this context, means antimicrobial and/or antifungal activity that is at least 50% of the anti-microbial and/or antifungal activity of the sodium salt(s) of EDTA and Ethanol composition in aqueous solution with a concentration of 0.12% (w/v) sodium salt(s) of EDTA that comprises tetra-sodium EDTA and 6.25% (v/v) Ethanol at a pH of 10.5+/-0.5.

Further, embodiments of the invention include compositions wherein the antimicrobial and/or antifungal activity is at least 60%, or 70% or 80% or 90% of the anti-microbial and/or antifungal activity of sodium salt(s) of EDTA that comprises tetra-sodium EDTA and Ethanol composition in aqueous solution with a concentration of 4% (w/v) sodium salt(s) of EDTA that comprises tetra-sodium EDTA and 12.5% (v/v) Ethanol at a pH of 10.5+/-0.5.

Further, embodiments of the invention include compositions wherein the antimicrobial and/or antifungal activity is at least 90% or higher of the antimicrobial and/or antifungal activity of sodium salt(s) of EDTA and Ethanol composition in aqueous solution with a concentration of 1-4% (w/v) sodium salt(s) of EDTA, 10-25% (v/v) Ethanol and Mannitol 0.5 to 1% (w/v) at a pH range of 7 to 11. Also disclosed are methods of using the compositions. In an embodiment, a method for disinfecting a catheter is disclosed, the method comprising: introducing a composition into an interior lumen of the catheter, wherein the composition is as defined in claim 1.

In another aspect, a method for disinfecting, maintaining catheter patency, and an International Normalized Ratio (INR) of at least 0.5 points higher than that of the INR of 10 U/ml heparin is disclosed, said method comprising of introducing a composition into an interior lumen of the catheter, wherein the composition is as defined in claim 1.

In some embodiments, disinfectant compositions of the present disclosure comprising sodium salt(s) of EDTA, Ethanol and D-Mannitol having specified concentration(s), at specified pH ranges, are substantially free from other active substances having substantial antimicrobial and/or anti-fungal activity.

In solution, the sodium salt(s) of EDTA, Ethanol and D-Mannitol are dissolved in a solvent, which is an aqueous solution, such as water or saline, or another biocompatible solution in which the sodium salt(s) of EDTA, Ethanol and D-Mannitol are soluble, or a combination thereof.

Sodium salt(s) of EDTA, Ethanol and D-Mannitol solutions of the present disclosure are preferably provided in a sterile and non-pyrogenic form and may be packaged in any convenient fashion. In some embodiments, disinfectant sodium salt(s) of EDTA, Ethanol and D-Mannitol compositions of the present disclosure may be provided in connection with or as part of a medical device, such as in a pre-filled syringe or another medical device. The compositions may be prepared under sterile, aseptic conditions, or they may be sterilized following preparation and/or packaging using any of a variety of suitable sterilization techniques. Single use vials, syringes or containers of sodium salt(s) of EDTA, Ethanol and D-Mannitol solutions may be provided. Multiple use vials, syringes or containers may also be provided. Systems of the present disclosure include such vials, syringes or containers containing the sodium salt(s) of EDTA, Ethanol and D-Mannitol solutions of the present disclosure.

Compositions comprising Ethanol have a well-established safety profile in connection with medical usage and administration to humans. Ethanol is present, in combination with other components, in many solutions used in medical and human health applications. It has been established as safe for human use, both in vitro and in vivo. Ethanol is readily available at a reasonable cost, and is stable over time in solution.

Mannitol is utilized in various medical applications, primarily as bulking agents. It is used either singly or in combination with other agents. It has been established as safe for human use, both in-vitro and in-vivo. It is readily available at a reasonable cost, and is stable over time in solution.

Formulation and production of disinfectant compositions of the present disclosure are generally straightforward. In one embodiment, desired disinfectant compositions of the present disclosure are formulated by dissolving the sodium salt(s) of EDTA, Ethanol and D-Mannitol in an aqueous solvent, such as distilled water or phosphate buffer saline, to the desired concentration and adjusting the solution pH to the desired level. In alternative embodiments, desired disinfectant compositions of the present disclosure are formulated by dissolving the sodium salt(s) of EDTA, Ethanol and D-Mannitol in a solvent to provide a concentrated, solubilized solution, and additional solvents or components may then be added.

Disinfectant compositions of the present disclosure are also useful for many other applications. Sodium salt(s) of EDTA, Ethanol and D-Mannitol solutions may be used as disinfectant solutions for soaking, or rinsing, or contacting medical, dental and veterinary surfaces and objects. Sodium salt(s) of EDTA, Ethanol and D-Mannitol solutions of the present disclosure may be used, for example, for storing and/or disinfectant contact lenses and other optical devices; for storing and/or disinfectant dental devices such as dentures, bridges, retainers, tooth brushes, and the like, and for storing and/or disinfectant medical and dental and veterinary devices and instruments. In these applications, the devices or surfaces may be contacted with sodium salt(s) of EDTA, Ethanol and Mannitol solutions of the present disclosure for a time sufficient to substantially eliminate microbial and/or fungicidal infections, or devices and surfaces may be soaked in sodium salt(s) of EDTA, Ethanol and D-Mannitol solutions for a desired time period. Sodium salt(s) of EDTA, Ethanol and D-Mannitol compositions of the present disclosure may additionally be used to disinfect water and other fluid supply lines. Disinfection of fluid supply lines may be accomplished by intermittently flushing the lines with sodium salt(s) of EDTA, Ethanol and D-Mannitol compositions of the present disclosure. Similarly, sodium salt(s) of EDTA, Ethanol and D-Mannitol compositions of the present disclosure may be used to eradicate bio-films and microbial (including some virus and protozoa) and fungal populations in water supply and storage devices.

Conduits may be treated with sodium salt(s) of EDTA, Ethanol and D-Mannitol solutions as a preventative disinfectant or as treatment following potential fungal or bacterial infection. The treatment of conduits can include locking, flushing, coating, or aerosol doses of the sodium salt(s) of EDTA, Ethanol and D-Mannitol solution. Examples of conduits that may be treated using the sodium salt(s) of EDTA, Ethanol and D-Mannitol solution include, but not limited to, water lines in dental or medical offices, lines carrying sterile fluids, catheters or ports that carry blood and/or other fluids into and out of the body, industrial water supply lines which develop large bio-film populations which effect the efficient flow of fluids as well as contaminating the fluids passing through the line, and airway support devices. Other examples include, but not limited to, consumption such as drink dispensers and food packaging. Conduits treated by the sodium salt(s) of EDTA, Ethanol and Mannitol solution are typically made of plastic, but the principles of the present disclosure may be applied to conduit device made of any material such as metal that delivers or carries fluid.

A sodium salt(s) of EDTA, Ethanol and Mannitol solution can be used in treatment of topical infections, including but not limited to skin, ear, anal, mouth, and vulvo/vaginal sites.

A sodium salt(s) of EDTA, Ethanol and Mannitol solution can be used in as an effective disinfectant for surfaces and equipment in industrial, medical, and household applications. A typical infected system would include the walls, floors, and commode in a lavatory. The delivery system will typically comprise a solvent and tools that allow flushing, locking, wiping, soaking, fogging, or coating of the surface defining the infected system.

A sodium salt(s) of EDTA, Ethanol and D-Mannitol solution can be used as an effective decontamination disinfectant for medical instruments and devices, dental (both consumer and professional) instruments and devices, and/or veterinary instruments and devices. A typical example would be a soak for disinfecting toothbrushes.

A sodium salt(s) of EDTA, Ethanol and D-Mannitol solution can be used as an effective disinfectant solution for optical contact lenses.

A sodium salt(s) of EDTA, Ethanol and D-Mannitol solution can be used as a treatment for catheters defining an infected system. The sodium salt(s) of EDTA, Ethanol and D-Mannitol solution may inhibit microbe colonization by treating the catheter with the solution at the prescribed concentration using a liquid lock prior to and in between infusions and/or by surface coating of catheter devices. A further application is the treatment of colonized or infected catheters by use of a liquid lock containing the sodium salt(s) of EDTA, Ethanol and D-Mannitol solution in the preferred concentration and pH.

Typically, the sodium salt(s) of EDTA, Ethanol and D-Mannitol solution, when used to treat catheters, are dissolved in water as a carrier, although other carriers may be used. Substances such as thrombolytics, sodium, alcohol, or reagents may also be added to the basic water/sodium salt(s) of EDTA, Ethanol and D-Mannitol solution.

**MINIMUM INHIBITORY CONCENTRATION (MIC) - REFERENCE EXPERIMENTS**

Experiments were conducted to assess the minimum inhibitory concentration (MIC) both for sodium salt of EDTA that comprises tetra-sodium EDTA and Ethanol. This was conducted primarily, as the name suggests, to evaluate the minimum concentration where these reagents inhibit the growth of microorganisms. The method used was a variant of National Committee for Clinical Laboratory Standards (NCCLS) macro-dilution procedure. In this experiment multiple concentrations of the solutions are prepared and exposed to the microorganisms for a period of 24 hours. Following incubation the growth is visually observed against the light. If the solutions exhibit turbidity / haziness it indicates growth. Clear solution indicates no growth. The minimum concentration at which no growth is observed is defined as the minimum inhibitory concentration (MIC).

The following solutions were prepared to evaluate the MIC values of sodium salt of EDTA that comprises tetra-sodium EDTA (TEDTA) and Ethanol. The reagents used were of Titriplex III GR [Ethylenedinitrilotetraacetic acid disodium salt (dihydrate)], Catalogue No 60841801001730, Lot No. QD3Q630747 Merck Specialties Private Limited; Ethanol Absolute, Catalogue No XK-13-011-00009, Lot No 20150125, Changshu Yangyuan Chemical, China.

| TEDTA (CONC. WT%) | ETHANOL (CONC. VOL%) |
|---|---|
| 0.015 | 3.13 |
| 0.030 | 6.25 |
| 0.060 | 12.50 |
| 0.120 | 25.00 |
| 0.240 | 50.00 |

## SYNERGY REFERENCE EXPERIMENTS

Two sets of experiments were conducted to show an unexpected synergism of the disinfectant activity of a composition that includes both a sodium salt of EDTA that comprises tetra-sodium EDTA and Ethanol.

The first set of experiments conducted, were screening experiments using checkerboard titration strategy to assess if the combinations fall within a range having an Fractional Inhibitory Concentration (FIC) index value of ≤1. The method used was a NCCLS macro-dilution procedure.

The second set of experiments conducted, were "Rate Kill" assay. A rate kill assay can confirm whether combinations are synergistic or not. In these assays the formulations are first exposed to organisms for a desired time (for the current formulations readings were taken at 1, 3 and 24 hours). Then a sample of the organisms and formulation mixture is serially diluted and plated to assess the log reduction. The organisms are allowed to grow and are checked for growth/log reduction after 24 hrs. The log reduction values obtained for individual components are compared with the combinations. Any combination(s) having >2 log reduction when compared with the most active compound used in the combination at any time point tested are labeled as synergistic (Comparison of methods for assessing synergic antibiotic interactions, M.L Mackay, K Miline, I. M. Gould, International journal of antimicrobial agents, 15 (2000) 125-129).

Based on the strategy stated above, experiments were conducted to investigate the effect of Ethanol on the antimicrobial activity of tetra-sodium EDTA. Reagents used were Titriplex III GR [Ethylenedinitrilotetraacetic acid disodium salt (dihydrate)], Catalogue No 60841801001730, Lot No. QD3Q630747 Merck Specialties Private Limited; Ethanol Absolute, Catalogue No XK-13-011-00009, Lot No 20150125, Changshu Yangyuan Chemical, China.

## CHECKERBOARD TITRATION REFERENCE EXPERIMENT - (S. aureus)

The Checkerboard Titration method was used to assess the interactions between TEDTA and Ethanol. The Checkerboard Titration method is a frequently used technique where, for example, each agent (TEDTA and Ethanol) was tested at multiple dilutions lower than the MIC. During this experiment, TEDTA and Ethanol were tested in the combinations to assess if the combinations have a Fractional Inhibitory Concentration (FIC) index of ≤1. The following concentrations were tested:

| COMBINATION | CONCENTRATION TEDTA (WT%) | CONCENTRATION ETHANOL (VOL%) |
|---|---|---|
| 0.5 MIC + 0.5 MIC | 0.120 | 12.5 |
| 0.25 MIC + 0.25 MIC | 0.060 | 6.25 |
| 0.125 MIC + 0.125 MIC | 0.030 | 3.125 |

Fractional Inhibitory Concentration (FIC) is defined as the MIC of the compound in combination divided by the MIC of the compound alone. If the FIC index is ≤0.5, the combination is interpreted to be synergistic; <1 but >0.5 - as partially synergistic; =1 as additive; >1 but <4 as indifferent; and ≥4 as antagonistic. In order to calculate the FIC index the following calculations are performed for compounds A and B:

$$\text{FIC-A} = (\text{MIC of A in combination})/(\text{MIC of A alone})$$

$$\text{FIC-B} = (\text{MIC of B in combination})/(\text{MIC of B alone})$$

$$\text{FIC-combination} = \text{FIC-A} + \text{FIC-B}$$

The MIC-TEDTA (MIC of TEDTA in combination with Ethanol), a minimum concentration of TEDTA, while in combination with Ethanol, which inhibited the growth of S. aureus in Mueller Hinton Broth (MHB) was found. In order to determine the MIC-Ethanol (MIC of Ethanol in combination with TEDTA), a minimum concentration of Ethanol, while in combination with TEDTA, which inhibited the growth of S. aureus in MHB was found. As per experiments conducted above, the MIC-TEDTA is 0.06 % (w/v) for S. aureus and the MIC-ETH is 6.25 % (w/v) for S. aureus. See table 4 for results.
Thus, the FIC-TEDTA is 0.06/0.24, which equals 0.25. The FIC-Ethanol is 6.25/25, which equals 0.25. Thus, the FIC-combination is 0.25+0.25, which equals 0.50. Accordingly, the combination of TEDTA and Ethanol unexpectedly has synergistic results. That is, embodiments of the combination of TEDTA and Ethanol provides results that are, unexpectedly, greater than the total effects of each agent operating by itself. This synergistic effect of embodiments of the combination of TEDTA and Ethanol may provide enhanced activity against bio-films. Without being bound to theory, it is suspected that TEDTA will degrade the bio-film, thus making the sessile microorganisms planktonic, allowing Ethanol to act against the more susceptible planktonic microorganisms. See table 4 for results.

**CHECKERBOARD TITRATION REFERENCE EXPERIMENT - C. albicans**

The following concentrations were tested:

| COMBINATION | CONCENTRATION TEDTA (WT%) | CONCENTRATION ETHANOL (VOL%) |
|---|---|---|
| 0.5 MIC + 0.5 MIC | 0.120 | 1.5625 |
| 0.25 MIC + 0.25 MIC | 0.060 | 0.78125 |
| 0.125 MIC + 0.125 MIC | 0.030 | 0.39062 |

The MIC-TEDTA (MIC of TEDTA in combination with Ethanol), a minimum concentration of TEDTA, while in combination with Ethanol, that inhibited the growth of C. albicans in MHB was found. In order to determine the MIC-Ethanol (MIC of Ethanol in combination with TEDTA), a minimum concentration of Ethanol, while in combination with TEDTA, that inhibited the growth of C. albicans in MHB was found. As per experiments conducted above, the MIC-TEDTA is 0.06 % (w/v) for C. albicans and the MIC-Ethanol is 0.781 % (v/v) for C. albicans. See table 5 for results.
Thus, the FIC-TEDTA is 0.06/0.24, which equals 0.25. The FIC-Ethanol is 0.781/3.125, which equals 0.25. Thus, the FIC-combination is 0.25+0.25, which equals 0.5. Accordingly, the combination of TEDTA and Ethanol unexpectedly has synergistic results. That is, embodiments of the combination of TEDTA and Ethanol provides results that are, unexpectedly, greater than the total effects of each agent operating by itself. This synergistic effect of embodiments of the combination of TEDTA and Ethanol may provide enhanced activity against bio-films. Without being bound to theory, it is suspected that TEDTA will degrade the bio-film, thus making the sessile microorganisms planktonic, allowing Ethanol to act against the more susceptible planktonic microorganisms.

**CHECKERBOARD TITRATION REFERENCE EXPERIMENT - P. aeruginosa**

The following concentrations were tested:

| COMBINATION | CONCENTRATION TEDTA (WT%) | CONCENTRATION ETHANOL (VOL%) |
|---|---|---|
| 0.48 MIC + 0.5 MIC | 0.120 | 3.125 |
| 0.24 MIC + 0.25 MIC | 0.060 | 1.5625 |
| 0.12 MIC + 0.125 MIC | 0.030 | 0.78125 |

The MIC-TEDTA (MIC of TEDTA in combination with Ethanol), a minimum concentration of TEDTA, while in combination with Ethanol, that inhibited the growth of P. aeruginosa in MHB was found. In order to determine the MIC-Ethanol (MIC of Ethanol in combination with TEDTA), a minimum concentration of Ethanol, while in combination with TEDTA, that inhibited the growth of P. aeruginosa in MHB was found. As per experiments conducted above, the MIC-TEDTA is 0.12 % (w/v) for P. aeruginosa and the MIC-Ethanol is 3.125 % (v/v) for P. aeruginosa. See table 6 for results.

Thus, the FIC-TEDTA is 0.12/0.25, which equals 0.48. The FIC-Ethanol is 3.125/6.25, which equals 0.5. Thus, the FIC-combination is 0.48+0.50, which equals 0.98. Accordingly, the combination of TEDTA and Ethanol unexpectedly has partially synergistic results. That is, embodiments of the combination of TEDTA and Ethanol provides results that are, unexpectedly, greater than the total effects of each agent operating by itself. This partially synergistic effect of embodiments of the combination of TEDTA and Ethanol may provide enhanced activity against bio-films. Without being bound to theory, it is suspected that TEDTA will degrade the bio-film, thus making the sessile microorganisms planktonic, allowing Ethanol to act against the more susceptible planktonic microorganisms.

## RATE KILL STUDY

This study was conducted in two parts to assess -

a) the synergy and
b) the effect of pH and D-Mannitol on the antimicrobial efficacy of the EDTA and ethanol based formulations.

## PART A (REFERENCE): SYNERGY ASSESSMENT RATE KILL ASSAY - S. aureus

The following solutions were prepared:

| COMPOSITION | WT% OR VOL% |
|---|---|
| TEDTA | 0.06 wt% |
| TEDTA | 0.12 wt% |
| TEDTA | 4.00 wt% |
| Ethanol | 6.25 vol% |
| Ethanol | 12.50 vol% |
| Ethanol | 25.00 vol% |
| TEDTA + Ethanol | 0.06 wt% + 6.25 vol% |
| TEDTA + Ethanol | 0.12 wt% + 12.50 vol% |
| TEDTA + Ethanol | 4.00 wt% + 12.50 vol% |
| TEDTA + Ethanol | 4.00 wt% + 25.00 vol% |

Each solution was then combined with S. aureus and the log reduction of the S. aureus was measured at 1, 3 and 24 hours. The difference in log reduction was 4.33 at 1 hour and 4.25 at 3 hours when EDTA + Ethanol combinations were compared with the most active component in the combination alone. See table 7 for results. Accordingly, the data shows that TEDTA and Ethanol solutions are synergistic. That is, embodiments of the combination of TEDTA and Ethanol provides results that are, unexpectedly, greater than the total effects of each agent operating by itself. This synergistic effect of embodiments of the combination of TEDTA and Ethanol is expected to provide enhanced activity against bio-films. Without being bound to theory, it is suspected that TEDTA salt(s) will degrade the bio-film, thus making the sessile microorganisms planktonic, allowing Ethanol to act against the more susceptible planktonic microorganisms.

## RATE KILL ASSAY - C. albicans

The following solutions were prepared:

| COMPOSITION | WT% OR VOL% |
|---|---|
| TEDTA | 0.06 wt% |

(continued)

| COMPOSITION | WT% OR VOL% |
|---|---|
| TEDTA | 0.12 wt% |
| TEDTA | 4.00 wt% |
| Ethanol | 0.78 vol% |
| Ethanol | 1.56 vol% |
| Ethanol | 12.50 vol% |
| Ethanol | 25.00 vol% |
| TEDTA + Ethanol | 0.06 wt% + 0.78 vol% |
| TEDTA + Ethanol | 0.12 wt% + 1.56 vol% |
| TEDTA + Ethanol | 4.00 wt% + 12.50 vol% |
| TEDTA + Ethanol | 4.00 wt% + 25.00 vol% |

Each solution was then combined with C. albicans and the log reduction of the C. albicans was measured at 1, 3 and 24 hours. The difference in log reduction was 2.90 at 1 hour for the 4 wt% TEDTA + 12.5 vol% Ethanol combination when compared with the most active ingredient i.e. 12.5 vol% Ethanol in the combination. See table 8 for results. Accordingly, the data shows that TEDTA and Ethanol solutions are synergistic. That is, embodiments of the combination of TEDTA and Ethanol provides results that are, unexpectedly, greater than the total effects of each agent operating by itself. This synergistic effect of embodiments of the combination of TEDTA and Ethanol is expected to provide enhanced activity against bio-films. Without being bound to theory, it is suspected that TEDTA salt(s) will degrade the bio-film, thus making the sessile microorganisms planktonic, allowing Ethanol to act against the more susceptible planktonic microorganisms.

## RATE KILL ASSAY - P. aeruginosa

The following solutions were prepared:

| COMPOSITION | WT% OR VOL% |
|---|---|
| TEDTA | 0.06 wt% |
| TEDTA | 0.12 wt% |
| TEDTA | 4.00 wt% |
| Ethanol | 1.56 vol% |
| Ethanol | 3.13 vol% |
| Ethanol | 12.50 vol% |
| Ethanol | 25.00 vol% |
| TEDTA + Ethanol | 0.06 wt% + 1.563 vol% |
| TEDTA + Ethanol | 0.12 wt% + 3.125 vol% |
| TEDTA + Ethanol | 4.00 wt% + 12.50 vol% |
| TEDTA + Ethanol | 4.000 wt% + 25.00 vol% |

Each solution was then combined with P. aeruginosa and the log reduction of the P. aeruginosa was measured at 1, 3 and 24 hours. The difference in log reduction was 4.07 at 1 hour and 2.78 at 3 hours for the 4 wt% TEDTA + 12.5 vol% Ethanol combination when compared with the most active ingredient i.e. 12.5 vol% Ethanol in the combination. See table 9 for results. Accordingly, the data shows that TEDTA and Ethanol solutions are synergistic. That is, embodiments of the combination of TEDTA and Ethanol provides results that are, unexpectedly, greater than the total effects of each agent operating by itself. This synergistic effect of embodiments of the combination of TEDTA and Ethanol is expected to provide enhanced activity against bio-films. Without being bound to theory, it is suspected that TEDTA salt(s) will degrade the bio-film, thus making the sessile microorganisms planktonic, allowing Ethanol to act against the more susceptible planktonic microorganisms.

The synergistic effect (rate kill assay) and partial/fully synergistic effect (checkerboard titration) provides significant, practical advantages for uses of embodiments of the combination of TEDTA and Ethanol. As noted, bio-films are a significant problem in a variety of fields. The bio-film protective substance, often referred to as extra-cellular polymeric substance (EPS), polysaccharide covering or glycocalyx, provides a protection to bio-films that are difficult to inhibit or eradicate. A solution that can inhibit or eradicate a bio-film is an important alternative. The proper use of antibiotics to eradicate a bio-film is costly, time consuming and may result in the development of antibiotic resistant bacterial strains, which cannot be effectively treated. Thus, embodiments of the present invention should prevent the overuse of broad-spectrum antibiotics and continued unnecessary catheter removal and replacement procedures.

**PART B: EFFECT pH & D-MANNITOL ADDITION ASSESSMENT RATE KILL ASSAY** -

The following solutions were prepared (in the following table the compositions in rows 1, 2, 5 and 6 are according to the claims, the compositions in rows 3, 4, 7 and 8 are reference compositions):

| COMPOSITION | WT% OR VOL% | pH |
|---|---|---|
| EDTA + Ethanol + D-Mannitol | 4.00 wt% + 25.00 vol% + 0.73 wt% | 7.5 |
| EDTA + Ethanol + D-Mannitol | 1.50 wt% + 12.50 vol% + 0.73 wt% | 7.5 |
| EDTA + Ethanol | 1.50 wt% + 12.50 vol% | 7.5 |
| D-Mannitol | 0.73 wt% | 7.5 |
| EDTA + Ethanol + D-Mannitol | 4.00 wt% + 25.00 vol% + 0.73 wt% | 11.0 |
| EDTA + Ethanol + D-Mannitol | 1.50 wt% + 12.50 vol% + 0.73 wt% | 11.0 |
| EDTA + Ethanol | 1.50 wt% + 12.50 vol% | 11.0 |
| D-Mannitol | 0.73 wt% | 11.0 |

Each solution was then combined with S. aureus and the log reduction of the S. aureus was measured at 1, 3 and 24 hours. The data shows enhancement in antimicrobial efficacy (>2 logs at 1 & 3 hrs time point) with pH increase from 7.5 to 11 for EDTA + Ethanol + D-Mannitol. Further, D-Mannitol exhibits >4 log reduction, which is unexpectedly high both at pH 7.5 & 11. See table 10 for results.

Each solution was then combined with C. albicans and the log reduction of the C. albicans was measured at 1, 3 and 24 hours. The data exhibits that with increase in pH, the antimicrobial efficacy remains similar for EDTA + Ethanol + D-Mannitol. This particular finding would be extremely beneficial since it would allow the formulation to be used at physiological pH without compromising the activity. Further, D-Mannitol exhibits >5 log reduction, which is unexpectedly high both at pH 7.5 & 11. See table 11 for results.

Each solution was then combined with P. aeruginosa and the log reduction of the P. aeruginosa was measured at 1, 3 and 24 hours. The data exhibits that with increase in pH, the antimicrobial efficacy remains similar for EDTA + Ethanol + D-Mannitol. This particular finding would be extremely beneficial since it would allow the formulation to be used at physiological pH without compromising the activity. Further, D-Mannitol exhibits >5 log reduction, which is unexpectedly high, both at pH 7.5 & 11. See table 12 for results.

Further, the embodiments of the combination of EDTA, Ethanol and D-Mannitol are expected to provide enhanced activity against bio-films. Without being bound to theory, it is suspected that D-Mannitol will enhance antimicrobial sensitivity of bio-films, while EDTA salt(s) will degrade the bio-film, thus making the sessile microorganisms planktonic, allowing Ethanol to act against the more susceptible planktonic microorganisms.

**PROTHROMBIN TIME (PT) EXPERIMENTS:**

Experiments were conducted to assess the prothrombin time (PT) both for sodium salt of EDTA that comprises tetra-sodium EDTA and Ethanol. In addition, combination solutions that comprise of tetra-sodium EDTA and Ethanol were also tested. This was conducted primarily, to evaluate to what extent the solutions delays the clotting time when compared with the citrated control.

The blood was drawn from healthy males in the age group of 18 to 40 years with no history of any kind of anticoagulant therapy etc. Blood samples were collected in plain tubes containing the solutions in a ratio of 1:10, usually 1.8 or 2.7 ml blood is mixed with 0.2 or 0.3 ml solution. Plasma was obtained from each venous sample by centrifugation. Following centrifugation the red cells were removed by carefully pipetting the layer off to avoid the platelet layer. PT was then measured by STA-Neoplastine CI Plus (Diagnostica Stago, Asnieres, France).

This study was conducted in two parts to assess the -

a) anticoagulant activity of EDTA, Ethanol and in Combination.
b) effects of pH and D-Mannitol on anticoagulant activity of EDTA and Ethanol based formulations.

## PART A (REFERENCE)

Following solutions were prepared for PT assessment:

| COMPOSITION | WT% OR VOL% |
| --- | --- |
| TEDTA | 4.0 wt% |
| Ethanol | 12.5 vol% |
| Ethanol | 25.0 vol% |
| TEDTA + Ethanol | 4.0 wt% + 12.5 vol% |
| TEDTA + Ethanol | 4.0 wt% + 25.0 vol% |

10 U/ml of Heparin solution sample were provided by the medical practitioners. The manufacturer was Troikka Pharmaceuticals.

The above solutions were assessed for PT. See table 13 for results. From the table it is evident that the formulations containing TEDTA and Ethanol have higher PT when compared with heparin alone. This clearly indicates the anticoagulant capability of the formulations.

## PART B

In the following table the compositions in rows 1, 2, 5 and 6 are according to the claims, the compositions in rows 3, 4, 7 and 8 are reference compositions.

| COMPOSITION | WT% OR VOL% | pH |
| --- | --- | --- |
| EDTA + Ethanol + D-Mannitol | 4.00 wt% + 25.00 vol% + 0.73 wt% | 7.5 |
| EDTA + Ethanol + D-Mannitol | 1.50 wt% + 12.50 vol% + 0.73 wt% | 7.5 |
| EDTA + Ethanol | 1.50 wt% + 12.50 vol% | 7.5 |
| D-Mannitol | 0.73 wt% | 7.5 |
| EDTA + Ethanol + D-Mannitol | 4.00 wt% + 25.00 vol% + 0.73 wt% | 11.0 |
| EDTA + Ethanol + D-Mannitol | 1.50 wt% + 12.50 vol% + 0.73 wt% | 11.0 |
| EDTA + Ethanol | 1.50 wt% + 12.50 vol% | 11.0 |
| D-Mannitol | 0.73 wt% | 11.0 |

The above solutions were assessed for PT. See table 14 for results. From the table it is evident that a) the lower pH formulations have slightly better anticoagulant activity, b) addition of D-Mannitol helps in maintaining the PT for EDTA and Ethanol based formulations. In addition, it apparent that the formulations containing EDTA, Ethanol and D-Mannitol have higher PT when compared with heparin alone (see table 13). This clearly indicates the anticoagulant capability of the formulations. In the following tables 1-14, the data relative to compositions according to the invention are: samples 1, 2, 5 and 6 in tables 10-12, and rows 2, 3, 6 and 7 in table 14; the remaining data relate to reference examples.

**TABLE 1**

| MINIMUM INHIBITORY CONCENTRATION (MIC) VIA MACRO-DILUTION METHOD - RESULTS MICROORGANISM - S. aureus | | | | | | | |
|---|---|---|---|---|---|---|---|
| TEDTA (CONC WT%) | GROWTH | GROWTH | GROWTH | ETHANOL (CONC VOL%) | GROWTH | GROWTH | GROWTH |
| 0.015 | +VE | +VE | +VE | 3.13 | +VE | +VE | +VE |
| 0.030 | +VE | +VE | +VE | 6.25 | +VE | +VE | +VE |
| 0.060 | +VE | +VE | +VE | 12.50 | +VE | +VE | +VE |
| 0.120 | +VE | +VE | +VE | **25.00** | **-VE** | **-VE** | **-VE** |
| **0.240** | **-VE** | **-VE** | **-VE** | 50.00 | -VE | -VE | -VE |
| | | | | | | | |
| POSITIVE CONTROL | +VE | +VE | +VE | | | | |
| NEGATIVE CONTROL | -VE | -VE | -VE | | | | |
| TEDTA - MIC 0.24 WT% <br> ETHANOL - MIC 25 VOL% | | | | | | | |

**TABLE 2**

| MINIMUM INHIBITORY CONCENTRATION (MIC) VIA MACRO-DILUTION METHOD - RESULTS MICROORGANISM - C. albicans | | | | | | | |
|---|---|---|---|---|---|---|---|
| TEDTA (CONC WT%) | GROWTH | GROWTH | GROWTH | ETHANOL (CONC VOL%) | GROWTH | GROWTH | GROWTH |
| 0.015 | +VE | +VE | +VE | **3.125** | **-VE** | **-VE** | **-VE** |
| 0.030 | +VE | +VE | +VE | 6.25 | -VE | -VE | -VE |
| 0.060 | +VE | +VE | +VE | 12.50 | -VE | -VE | -VE |
| 0.120 | +VE | +VE | +VE | 25.00 | -VE | -VE | -VE |
| **0.240** | **-VE** | **-VE** | **-VE** | 50.00 | -VE | -VE | -VE |
| | | | | | | | |
| POSITIVE CONTROL | +VE | +VE | +VE | | | | |
| NEGATIVE CONTROL | -VE | -VE | -VE | | | | |
| TEDTA- MIC 0.24 WT% <br> ETHANOL - MIC 3.125 VOL% | | | | | | | |

**TABLE 3**

| MINIMUM INHIBITORY CONCENTRATION (MIC) VIA MACRO-DILUTION METHOD - RESULTS MICROORGANISM - P. aeruginosa | | | | | | | |
|---|---|---|---|---|---|---|---|
| TEDTA (CONC WT%) | GROWTH | GROWTH | GROWTH | ETHANOL (CONC VOL%) | GROWTH | GROWTH | GROWTH |
| 0.125 | +VE | +VE | +VE | 3.13 | +VE | +VE | +VE |
| **0.250** | **-VE** | **-VE** | **-VE** | **6.25** | **-VE** | **-VE** | **-VE** |
| 0.500 | -VE | -VE | -VE | 12.50 | -VE | -VE | -VE |
| 1.000 | -VE | -VE | -VE | 25.00 | -VE | -VE | -VE |
| 1.500 | -VE | -VE | -VE | 50.00 | -VE | -VE | -VE |
| | | | | | | | |
| POSITIVE CONTROL | +VE | +VE | +VE | | | | |
| NEGATIVE CONTROL | -VE | -VE | -VE | | | | |
| TEDTA - MIC 0.25 WT%<br>ETHANOL - MIC 6.25 VOL% | | | | | | | |

**TABLE 4**

| SYNERGY EVALUATION STUDY - RESULTS | | | | | | | | | |
|---|---|---|---|---|---|---|---|---|---|
| STRATEGY - CHECKERBOARD TITRATION; METHOD - MACRO DILUTION; MICROORGANISM - S. aureus | | | | | | | | | |
| TEDTA (CONC WT%) | MIC | GROWTH | ETHANOL (CONC VOL%) | MIC | GROWTH | TEDTA (CONC WT%) | ETHANOL (CONC VOL%) | FIC | GROWTH |
| IND. | | | IND. | | | COMB | | | |
| 0.24 | 1.00 | **-VE** | 25.000 | 1.00 | **-VE** | | | | |
| 0.12 | 0.50 | **+VE** | 12.500 | 0.50 | **+VE** | 0.12 | 12.500 | 1.00 | **-VE** |
| 0.06 | 0.25 | **+VE** | 6.250 | 0.25 | **+VE** | 0.06 | 6.250 | 0.50 | **-VE** |
| 0.03 | 0.13 | **N/D** | 3.125 | 0.13 | **N/D** | 0.03 | 3.125 | 0.25 | **+VE** |

FIC = 0.50 - HENCE TEDTA + ETHANOL BASED COMBINATION SOLUTIONS HAVE SYNERGISTIC ACTION AGAINST S. aureus -VE - NO GROWTH +VE - GROWTH N/D - NOT DONE IND - INDIVIDUAL REAGENTS COMB - COMBINATION SOLUTION FIC - FRACTIONAL INHIBITORY CONCENTRATION FIC = 1 (ADDITIVE); FIC = <1 BUT >0.5 PARTIAL SYNERGY; FIC <0.5 SYNERGY

**TABLE 5**

| SYNERGY EVALUATION STUDY - RESULTS | | | | | | | | | |
|---|---|---|---|---|---|---|---|---|---|
| STRATEGY - CHECKERBOARD TITRATION; METHOD - MACRO DILUTION; MICROORGANISM - C. albicans | | | | | | | | | |
| TEDTA (CONC WT%) | MIC | GROWTH | ETHANOL (CONC VOL%) | MIC | GROWTH | TEDTA (CONC WT%) | ETHANOL (CONC VOL%) | FIC | GROWTH |
| IND | | | IND | | | COMB | | | |
| 0.24 | 1.00 | -VE | 3.125 | 1.00 | -VE | | | | |
| 0.12 | 0.50 | -VE | 1.563 | 0.50 | +VE | 0.12 | 1.563 | 1.00 | -VE |
| 0.06 | 0.25 | -VE | 0.781 | 0.25 | +VE | 0.06 | 0.781 | 0.50 | -VE |
| 0.03 | 0.13 | N/D | 0.391 | 0.13 | N/D | 0.03 | 0.391 | 0.25 | +VE |

FIC = 0.50 - HENCE TEDTA + ETHANOL BASED COMBINATION SOLUTIONS HAVE SYNERGISTIC ACTION AGAINS albicans
-VE - NO GROWTH
+VE - GROWTH
N/D - NOT DONE
IND - INDIVIDUAL REAGENTS
COMB - COMBINATION SOLUTION
FIC - FRACTIONAL INHIBITORY CONCENTRATION
FIC = 1 (ADDITIVE); FIC = <1 BUT >0.5 PARTIAL SYNERGY; FIC <0.5 SYNERGY

**TABLE 6**

| SYNERGY EVALUATION STUDY - RESULTS | | | | | | | | | |
|---|---|---|---|---|---|---|---|---|---|
| STRATEGY - CHECKERBOARD TITRATION; METHOD - MACRO DILUTION; MICROORGANISM - P. aeruginosa | | | | | | | | | |
| TEDTA (CONC | MIC WT%) | GROWTH | ETHANOL (CONC VOL%) | MIC | GROWTH | TEDTA (CONC WT%) | ETHANOL (CONC VOL%) | FIC | GROWTH |
| IND. | | | IND. | | | COMB | | | |
| 0.24 | 0.96 | **-VE** | 6.250 | 1.00 | **-VE** | | | | |
| 0.12 | 0.48 | **+VE** | 3.125 | 0.50 | **+VE** | 0.12 | 3.125 | 0.98 | **-VE** |
| 0.06 | 0.24 | **+VE** | 1.563 | 0.25 | **+VE** | 0.06 | 1.563 | 0.49 | **+VE** |
| | | | | | | 0.03 | 0.781 | 0.245 | **+VE** |

FIC = 0.98 - HENCE TEDTA + ETHANOL BASED COMBINATION SOLUTIONS HAVE PARTIALLY SYNERGISTIC ACTION AGAINST P. aeruginosa
-VE - NO GROWTH
+VE - GROWTH
N/D - NOT DONE
IND - INDIVIDUAL REAGENTS
COMB - COMBINATION SOLUTION
FIC - FRACTIONAL INHIBITORY CONCENTRATION
FIC = 1 (ADDITIVE); FIC = <1 BUT >0.5 PARTIAL SYNERGY; FIC <0.5 SYNERGY

**S YNERGY EVALUATION STUDY - RESULTS**
**STRATEGY - RATE KILL; METHOD - PLATE COUNT; MICROORGANISM - S. aureus**

TABLE 7

| TIME | SOLN CODE | TEDTA (CONC WT%) (IND) | LOG RED | SOLN CODE | EtOH (CONC VOL%) (IND) | LOG RED | SOLN CODE | TEDTA (CONC WT%) (COMB) | ETHANOL (CONC VOL%) | LOG RED | COMB-IND | DIFF IN LOG RED |
|---|---|---|---|---|---|---|---|---|---|---|---|---|
| 1 HR | 1 | 0.06 | 3.60 | 4 | 6.25 | 3.58 | 7 | 0.06 | 6.25 | 3.60 | | |
|  | 2 | 0.12 | 3.53 | 5 | 12.50 | 3.67 | 8 | 0.12 | 12.50 | 3.67 | | |
|  | 3 | 4.0 | 3.74 | 6 | 25.00 | 8.00 | 9 | 4.0 | 12.50 | 8.00 | "9-5" | 4.33 |
|  |  |  |  |  |  |  | 10 | 4.0 | 25.00 | 8.00 | | |
| 3 HR | 1 | 0.06 | 3.55 | 4 | 6.25 | 3.62 | 7 | 0.06 | 6.25 | 3.70 | | |
|  | 2 | 0.12 | 3.51 | 5 | 12.50 | 3.65 | 8 | 0.12 | 12.50 | 4.03 | | |
|  | 3 | 4.0 | 4.02 | 6 | 25.00 | 7.90 | 9 | 4.0 | 12.50 | 7.90 | "9-5" | 4.25 |
|  |  |  |  |  |  |  | 10 | 4.0 | 25.00 | 7.90 | | |
| 24 HR | 1 | 0.06 | 3.06 | 4 | 6.25 | 4.15 | 7 | 0.06 | 6.25 | 4.22 | | |
|  | 2 | 0.12 | 3.00 | 5 | 12.50 | 7.30 | 8 | 0.12 | 12.50 | 7.30 | | |
|  | 3 | 4.0 | 7.30 | 6 | 25.00 | 7.30 | 9 | 4.0 | 12.50 | 7.30 | | |
|  |  |  |  |  |  |  | 10 | 4.0 | 25.00 | 7.30 | | |

COMBINATION IS SYNERGISTIC IF THE DIFFERENCE IN LOG REDUCTION IS GREATER THAN 2 LOGS WHEN COMBINATION IS COMPARED WITH THE MOST ACTIVE COMPONENT.
FROM THE ABOVE DATA IT IS EVIDENT THAT THE TEDTA + ETHANOL COMBINATION HAS SYNERGISTIC ACTION AGAINST S. aureus.

## TABLE 8

| | | TEDTA (CONC WT%) | | | EtOH (CONC VOL%) | | | TEDTA (CONC WT%) | EtOH (CONC VOL%) | | | DIFF IN LOG RED |
|---|---|---|---|---|---|---|---|---|---|---|---|---|
| **S YNERGY EVALUATION STUDY - RESULTS** <br> **STRATEGY - RATE KILL; METHOD - PLATE COUNT; MICROORGANISM - C. albicans** | | | | | | | | | | | | |
| **TIME** | **SOLN CODE** | **TEDTA (CONC WT%)** | **LOG RED** | **SOLN CODE** | **EtOH (CONC VOL%)** | **LOG RED** | **SOLN CODE** | **TEDTA (CONC WT%)** | **EtOH (CONC VOL%)** | **LOG RED** | **COMB-IND** | **DIFF IN LOG RED** |
| | | (IND) | | | (IND) | | | | (COMB) | | | |
| 1 HR | 1 | 0.06 | 3.44 | 4 | 0.78 | 3.43 | 8 | 0.06 | 0.78 | 3.48 | | |
| | 2 | 0.12 | 3.46 | 5 | 1.56 | 3.45 | 9 | 0.12 | 1.56 | 3.50 | | |
| | 3 | 4.0 | 3.92 | 6 | 12.50 | 5.00 | 10 | 4.0 | 12.50 | 7.90 | "10-6" | **2.90** |
| | | | | 7 | 25.00 | 7.90 | 11 | 4.0 | 25.00 | 7.90 | | |
| 3 HR | 1 | 0.06 | 3.48 | 4 | 0.78 | 3.47 | 8 | 0.06 | 0.78 | 3.60 | | |
| | 2 | 0.12 | 3.51 | 5 | 1.56 | 3.49 | 9 | 0.12 | 1.56 | 3.64 | | |
| | 3 | 4.0 | 7.90 | 6 | 12.50 | 7.90 | 10 | 4.0 | 12.50 | 7.90 | | |
| | | | | 7 | 25.00 | 7.90 | 11 | 4.0 | 25.00 | 7.90 | | |
| 24 HR | 1 | 0.06 | 3.32 | 4 | 0.78 | 3.22 | 8 | 0.06 | 0.78 | 7.60 | | |
| | 2 | 0.12 | 3.38 | 5 | 1.56 | 3.28 | 9 | 0.12 | 1.56 | 7.60 | | |
| | 3 | 4.0 | 7.60 | 6 | 12.50 | 7.60 | 10 | 4.0 | 12.50 | 7.60 | | |
| | | | | 7 | 25.00 | 7.60 | 11 | 4.00 | 25.00 | 7.60 | | |
| COMBINATION IS SYNERGISTIC IF THE DIFFERENCE IN LOG REDUCTION IS GREATER THAN 2 LOGS WHEN COMBINATION IS COMPARED WITH THE MOST ACTIVE COMPONENT. <br> FROM THE ABOVE DATA IT IS EVIDENT THAT THE TEDTA + ETHANOL COMBINATION HAS SYNERGISTIC ACTION AGAINST C. albicans. | | | | | | | | | | | | |

TABLE 9

**S YNERGY EVALUATION STUDY - RESULTS**
**STRATEGY - RATE KILL; METHOD - PLATE COUNT; MICRO ORGANISM - P. aeruginosa**

| TIME | SOLN CODE | TEDTA (CONC WT%) | LOG RED | SOLN CODE | EtOH (CONC VOL%) | LOG RED | SOLN CODE | TEDTA (CONC WT%) | EtOH (CONC VOL%) | LOG RED | COMB-IND | DIFF IN LOG RED |
|---|---|---|---|---|---|---|---|---|---|---|---|---|
| | | (IND) | | | (IND) | | | | (COMB) | | | |
| 1 HR | 1 | 0.06 | 3.75 | 4 | 1.56 | 3.76 | 8 | 0.06 | 1.563 | 5.12 | | |
| | 2 | 0.12 | 3.83 | 5 | 3.13 | 3.76 | 9 | 0.12 | 3.125 | 5.20 | | |
| | 3 | 4.0 | 4.23 | 6 | 12.50 | 4.13 | 10 | 4.0 | 12.50 | 8.20 | "10-6" | **4.07** |
| | | | | 7 | 25.00 | 8.20 | 11 | 4.0 | 25.00 | 8.20 | | |
| 3 HR | 1 | 0.06 | 3.89 | 4 | 1.56 | 3.65 | 8 | 0.06 | 1.563 | 6.08 | | |
| | 2 | 0.12 | 4.32 | 5 | 3.13 | 3.73 | 9 | 0.12 | 3.125 | 8.08 | | |
| | 3 | 4.0 | 5.12 | 6 | 12.50 | 5.30 | 10 | 4.0 | 12.50 | 8.08 | "10-6" | **2.78** |
| | | | | 7 | 25.00 | 8.08 | 11 | 4.0 | 25.00 | 8.08 | | |
| 24 HR | 1 | 0.06 | 4.32 | 4 | 1.56 | 3.64 | 8 | 0.06 | 1.563 | 7.90 | | |
| | 2 | 0.12 | 7.90 | 5 | 3.13 | 7.90 | 9 | 0.12 | 3.125 | 7.90 | | |
| | 3 | 4.0 | 7.90 | 6 | 12.50 | 7.90 | 10 | 4.0 | 12.50 | 7.90 | | |
| | | | | 7 | 25.00 | 7.90 | 11 | 4.0 | 25.00 | 7.90 | | |

COMBINATION IS SYNERGISTIC IF THE DIFFERENCE IN LOG REDUCTION IS GREATER THAN 2 LOGS WHEN COMBINATION IS COMPARED WITH THE MOST ACTIVE COMPONENT.
FROM THE ABOVE DATA IT IS EVIDENT THAT THE TEDTA + ETHANOL COMBINATION HAS SYNERGISTIC ACTION AGAINST P. aeruginosa.

**TABLE 10**

| TIME | SOLN CODE | EDTA (CONC WT%) | EtOH (CONC VOL%) | MAN(CONC WT%) | pH | LOG RED | LOG RED | AVG. LOG RED |
|---|---|---|---|---|---|---|---|---|
| | | pH AND D-MANNITOL ADDITION EFFECT EVALUATION STUDY - RESULTS STRATEGY - RATE KILL; METHOD - PLATE COUNT; MICRO ORGANISM - S. aureus | | | | | | |
| 1 HR | 1 | 4.00 | 25.00 | 0.73 | 7.5 | 5.03 | 5.05 | 5.04 |
| | 2 | 1.50 | 12.50 | 0.73 | 7.5 | 4.70 | 4.70 | 4.70 |
| | 3 | 1.50 | 12.50 | 0.00 | 7.5 | 4.49 | 4.49 | 4.49 |
| | 4 | 0.00 | 0.00 | 0.73 | 7.5 | 4.53 | 4.53 | 4.53 |
| | 5 | 4.00 | 25.00 | 0.73 | 11.0 | 7.58 | 7.58 | 7.58 |
| | 6 | 1.50 | 12.50 | 0.73 | 11.0 | 4.77 | 4.77 | 4.77 |
| | 7 | 1.50 | 12.50 | 0.00 | 11.0 | 4.59 | 4.58 | 4.59 |
| | 8 | 0.00 | 0.00 | 0.73 | 11.0 | 4.38 | 4.38 | 4.38 |
| 3 HR | 1 | 4.00 | 25.00 | 0.73 | 7.5 | 5.50 | 5.46 | 5.48 |
| | 2 | 1.50 | 12.50 | 0.73 | 7.5 | 4.95 | 4.95 | 4.95 |
| | 3 | 1.50 | 12.50 | 0.00 | 7.5 | 4.64 | 4.64 | 4.64 |
| | 4 | 0.00 | 0.00 | 0.73 | 7.5 | 4.67 | 4.66 | 4.66 |
| | 5 | 4.00 | 25.00 | 0.73 | 11.0 | 7.58 | 7.58 | 7.58 |
| | 6 | 1.50 | 12.50 | 0.73 | 11.0 | 6.97 | 7.27 | 7.12 |
| | 7 | 1.50 | 12.50 | 0.00 | 11.0 | 6.62 | 7.10 | 6.86 |
| | 8 | 0.00 | 0.00 | 0.73 | 11.0 | 4.47 | 4.47 | 4.47 |
| 24 HR | 1 | 4.00 | 25.00 | 0.73 | 7.5 | 5.65 | 5.68 | 5.67 |
| | 2 | 1.50 | 12.50 | 0.73 | 7.5 | 4.98 | 4.98 | 4.98 |
| | 3 | 1.50 | 12.50 | 0.00 | 7.5 | 4.70 | 4.70 | 4.70 |
| | 4 | 0.00 | 0.00 | 0.73 | 7.5 | 4.75 | 4.74 | 4.75 |
| | 5 | 4.00 | 25.00 | 0.73 | 11.0 | 7.53 | 7.53 | 7.53 |
| | 6 | 1.50 | 12.50 | 0.73 | 11.0 | 7.53 | 7.53 | 7.53 |
| | 7 | 1.50 | 12.50 | 0.00 | 11.0 | 7.53 | 7.53 | 7.53 |
| | 8 | 0.00 | 0.00 | 0.73 | 11.0 | 4.59 | 4.59 | 4.59 |
| WITH PH INCREASE LOG REDUCTION IS GREATER THAN 2 LOGS AT 1 & 3 HRS FOR 4% EDTA + 25% ETHANOL + 0.73% MANNITOL; D-MANNITOL EXHIBITS >4 LOG REDUCTION AGAINST S. aureus | | | | | | | | |

TABLE 11

| | | | | | | | | |
|---|---|---|---|---|---|---|---|---|
| | **pH AND D-MANNITOL ADDITION EFFECT EVALUATION STUDY - RESULTS**<br>**STRATEGY - RATE KILL; METHOD - PLATE COUNT; MICRO ORGANISM - C. albicans** |
| **TIME** | **SOLN CODE** | **EDTA (CONC WT%)** | **EtOH (CONC VOL%)** | **MAN (CONC WT%)** | **pH** | **LOG RED** | **LOG RED** | **AVG. LOG RED** |
| **1 HR** | 1 | 4.00 | 25.00 | 0.73 | 7.5 | 7.55 | 7.55 | 7.55 |
| | 2 | 1.50 | 12.50 | 0.73 | 7.5 | 5.71 | 5.69 | 5.70 |
| | 3 | 1.50 | 12.50 | 0.00 | 7.5 | 5.69 | 5.75 | 5.72 |
| | 4 | 0.00 | 0.00 | 0.73 | 7.5 | 5.37 | 5.39 | 5.38 |
| | 5 | 4.00 | 25.00 | 0.73 | 11.0 | 7.55 | 7.55 | 7.55 |
| | 6 | 1.50 | 12.50 | 0.73 | 11.0 | 5.88 | 5.84 | 5.86 |
| | 7 | 1.50 | 12.50 | 0.00 | 11.0 | 6.07 | 6.14 | 6.10 |
| | 8 | 0.00 | 0.00 | 0.73 | 11.0 | 5.27 | 5.27 | 5.27 |
| **3 HR** | 1 | 4.00 | 25.00 | 0.73 | 7.5 | 7.55 | 7.55 | 7.55 |
| | 2 | 1.50 | 12.50 | 0.73 | 7.5 | 6.21 | 6.21 | 6.21 |
| | 3 | 1.50 | 12.50 | 0.00 | 7.5 | 6.29 | 6.25 | 6.27 |
| | 4 | 0.00 | 0.00 | 0.73 | 7.5 | 5.55 | 5.57 | 5.56 |
| | 5 | 4.00 | 25.00 | 0.73 | 11.0 | 7.55 | 7.55 | 7.55 |
| | 6 | 1.50 | 12.50 | 0.73 | 11.0 | 6.51 | 6.51 | 6.51 |
| | 7 | 1.50 | 12.50 | 0.00 | 11.0 | 6.85 | 7.07 | 6.96 |
| | 8 | 0.00 | 0.00 | 0.73 | 11.0 | 5.42 | 5.40 | 5.41 |
| **24 HR** | 1 | 4.00 | 25.00 | 0.73 | 7.5 | 7.53 | 7.53 | 7.53 |
| | 2 | 1.50 | 12.50 | 0.73 | 7.5 | 7.53 | 7.53 | 7.53 |
| | 3 | 1.50 | 12.50 | 0.00 | 7.5 | 7.53 | 7.53 | 7.53 |
| | 4 | 0.00 | 0.00 | 0.73 | 7.5 | 5.76 | 5.74 | 5.75 |
| | 5 | 4.00 | 25.00 | 0.73 | 11.0 | 7.53 | 7.53 | 7.53 |
| | 6 | 1.50 | 12.50 | 0.73 | 11.0 | 7.53 | 7.53 | 7.53 |
| | 7 | 1.50 | 12.50 | 0.00 | 11.0 | 7.53 | 7.53 | 7.53 |
| | 8 | 0.00 | 0.00 | 0.73 | 11.0 | 5.55 | 5.58 | 5.56 |
| | WITH PH INCREASE ANTIMICROBIAL EFFICACY REMAINS SIMILAR FOR 4% EDTA + 25%ETHANOL + 0.73% D-MANNITOL D-MANNITOL EXHIBITS >5 LOG REDUCTION AGAINST C. albicans |

TABLE 12

| | | | | | | | | |
|---|---|---|---|---|---|---|---|---|
| **pH AND D-MANNITOL ADDITION EFFECT EVALUATION STUDY - RESULTS** | | | | | | | | |
| **STRATEGY - RATE KILL; METHOD - PLATE COUNT; MICRO ORGANISM - P. aeruginosa** | | | | | | | | |
| **TIME** | **SOLN CODE** | **EDTA (CONC WT%)** | **EtOH (CONC VOL%)** | **MAN (CONC WT%)** | **pH** | **LOG RED** | **LOG RED** | **AVG. LOG RED** |
| **1 HR** | 1 | 4.00 | 25.00 | 0.73 | 7.5 | 7.60 | 7.60 | 7.60 |
| | 2 | 1.50 | 12.50 | 0.73 | 7.5 | 7.60 | 7.60 | 7.60 |
| | 3 | 1.50 | 12.50 | 0.00 | 7.5 | 7.60 | 7.60 | 7.60 |
| | 4 | 0.00 | 0.00 | 0.73 | 7.5 | 5.41 | 5.39 | 5.40 |
| | 5 | 4.00 | 25.00 | 0.73 | 11.0 | 7.60 | 7.60 | 7.60 |
| | 6 | 1.50 | 12.50 | 0.73 | 11.0 | 6.56 | 6.56 | 6.56 |
| | 7 | 1.50 | 12.50 | 0.00 | 11.0 | 6.42 | 6.49 | 6.45 |
| | 8 | 0.00 | 0.00 | 0.73 | 11.0 | 5.42 | 5.42 | 5.42 |
| **3 HR** | 1 | 4.00 | 25.00 | 0.73 | 7.5 | 7.59 | 7.59 | 7.59 |
| | 2 | 1.50 | 12.50 | 0.73 | 7.5 | 7.59 | 7.59 | 7.59 |
| | 3 | 1.50 | 12.50 | 0.00 | 7.5 | 7.59 | 7.59 | 7.59 |
| | 4 | 0.00 | 0.00 | 0.73 | 7.5 | 5.73 | 5.75 | 5.74 |
| | 5 | 4.00 | 25.00 | 0.73 | 11.0 | 7.59 | 7.59 | 7.59 |
| | 6 | 1.50 | 12.50 | 0.73 | 11.0 | 7.59 | 7.59 | 7.59 |
| | 7 | 1.50 | 12.50 | 0.00 | 11.0 | 7.59 | 7.59 | 7.59 |
| | 8 | 0.00 | 0.00 | 0.73 | 11.0 | 5.74 | 5.80 | 5.77 |
| **24 HR** | 1 | 4.00 | 25.00 | 0.73 | 7.5 | 7.55 | 7.55 | 7.55 |
| | 2 | 1.50 | 12.50 | 0.73 | 7.5 | 7.55 | 7.55 | 7.55 |
| | 3 | 1.50 | 12.50 | 0.00 | 7.5 | 7.55 | 7.55 | 7.55 |
| | 4 | 0.00 | 0.00 | 0.73 | 7.5 | 6.51 | 6.44 | 6.47 |
| | 5 | 4.00 | 25.00 | 0.73 | 11.0 | 7.55 | 7.55 | 7.55 |
| | 6 | 1.50 | 12.50 | 0.73 | 11.0 | 7.55 | 7.55 | 7.55 |
| | 7 | 1.50 | 12.50 | 0.00 | 11.0 | 7.55 | 7.55 | 7.55 |
| | 8 | 0.00 | 0.00 | 0.73 | 11.0 | 6.55 | 6.25 | 6.40 |
| WITH PH INCREASE ANTIMICROBIAL EFFICACY REMAINS SIMILAR FOR 4% EDTA + 25%ETHANOL + 0.73% D-MANNITOL D-MANNITOL EXHIBITS >5 LOG REDUCTION AGAINST P. aeruginosa. | | | | | | | | |

TABLE 13

| | | | | | | |
|---|---|---|---|---|---|---|
| **PART A - PROTHROMBIN TIME (PT) STUDY - RESULTS** | | | | | | |
| **OBJECTIVE - TO ASSESS INTERACTION AMONG EDTA & ETHANOL FOR ANTICOAGULANT ACTIVITY** | | | | | | |
| **SAMPLES** | **INR-A** | **INR-B** | **INR-C** | **INR-D** | **AVERAGE INR** | **STD IN INR** |
| CITRATED CONTROL | 1.000 | 1.000 | 1.000 | 1.000 | 1.000 | 0.000 |
| HEPARIN SODIUM 10 IU/ML | 1.219 | 1.260 | 1.503 | 1.354 | 1.334 | 0.126 |
| 4 WT% TEDTA | 1.891 | 1.937 | 2.229 | 2.087 | 2.036 | 0.153 |

(continued)

| PART A - PROTHROMBIN TIME (PT) STUDY - RESULTS OBJECTIVE - TO ASSESS INTERACTION AMONG EDTA & ETHANOL FOR ANTICOAGULANT ACTIVITY | | | | | | |
|---|---|---|---|---|---|---|
| **SAMPLES** | **INR-A** | **INR-B** | **INR-C** | **INR-D** | **AVERAGE INR** | **STD IN INR** |
| 4 WT% TEDTA+25 VOL% ETHANOL | 2.052 | 2.041 | 2.409 | 2.470 | 2.243 | 0.228 |
| 4 WT% TEDTA+12.5 VOL% ETHANOL | 2.099 | 2.099 | 2.146 | 2.134 | 2.120 | 0.024 |
| 25 VOL% ETHANOL | 61.930 | 61.930 | 61.930 | NOT DONE | 61.930 | 0.000 |
| 12.5 VOL% ETHANOL | 61.930 | 61.930 | 61.930 | NOT DONE | 61.930 | 0.000 |
| THE ABOVE STUDIES WERE DONE ON TWO DIFFERENT DAYS AND USING DIFFERENT BLOOD FROM MALE POPULATION WITHIN THE AGE RANGE OF 18-40 YEARS. INR-A & INR-B - DURING FIRST ROUND WHILE INR-C & INR-D DURING SECOND ROUND. PT - PROTHROMBIN TIME CITRATED CONTROL - 13.8 SECS ASSAY CONTROL - 13.8 SECS INTERNATION STANDARDIZATION INDEX (ISI) - 1.34 INR = (SAMPLE PT / ASSAY CONTROL PT)^ISI FROM THE ABOVE RESULTS IT IS EVIDENT THAT 4% TEDTA+25% ETHANOL & 4% TEDTA+12.5% ETHANOL HAVE HIGHER INR WHEN COMPARED WITH 10 IU/ML HEPARIN. 4% TEDTA+25% ETHANOL HAVE HIGHER INR WHEN COMPARED WITH 10 IU/ML AS WELL AS 4% TEDTA+12.5% ETHANOL. | | | | | | |

## TABLE 14

| PART B - PROTHROMBIN TIME (PT) STUDY - RESULTS OBJECTIVE - TO ASSESS EFFECTS OF pH AND D-MANNITOL ADDITION TO EDTA & ETHANOL BASED FORMULATIONS | | | |
|---|---|---|---|
| **SAMPLES** | **WT% OR VOL%** | **pH** | **INR** |
| CITRATED CONTROL | N/A | N/A | 1.00 |
| EDTA + Ethanol + D-Mannitol | 4.00 wt% + 25.00 vol% + 0.73 wt% | 7.5 | 2.54 |
| EDTA + Ethanol + D-Mannitol | 1.50 wt% + 12.50 vol% + 0.73 wt% | 7.5 | 1.72 |
| EDTA + Ethanol | 1.50 wt% + 12.50 vol% | 7.5 | 1.74 |
| D-Mannitol | 0.73 wt% | 7.5 | N/A |
| EDTA + Ethanol + D-Mannitol | 4.00 wt% + 25.00 vol% + 0.73 wt% | 11.0 | 2.21 |
| EDTA + Ethanol + D-Mannitol | 1.50 wt% + 12.50 vol% + 0.73 wt% | 11.0 | 1.71 |
| EDTA + Ethanol | 1.50 wt% + 12.50 vol% | 11.0 | 1.64 |

(continued)

| PART B - PROTHROMBIN TIME (PT) STUDY - RESULTS | | | |
|---|---|---|---|
| **OBJECTIVE - TO ASSESS EFFECTS OF pH AND D-MANNITOL ADDITION TO EDTA & ETHANOL BASED FORMULATIONS** | | | |
| **SAMPLES** | **WT% OR VOL%** | **pH** | **INR** |
| D-Mannitol | 0.73 wt% | 11.0 | N/A |

THE ABOVE STUDY WAS DONE USING BLOOD FROM MALE POPULATION WITHIN THE AGE RANGE OF 18-40 YEARS.
PT - PROTHROMBIN TIME
CITRATED CONTROL - 11.9 SECS
ASSAY CONTROL - 11.9 SECS
INTERNATION STANDARDIZATION INDEX (ISI) - 1.24
INR = (SAMPLE PT / ASSAY CONTROL PT)^ISI
FROM THE ABOVE RESULTS IT IS EVIDENT THAT
A) pH HAS SLIGHT EFFECT - I.E. LOWER THE PH HIGHER IS THE PT TIME.
B) ADDITION OF MANNITOL HELPS IN MAINTAINING THE ANTICOGULANT ACTIVITY OF EDTA AND ETHANOL BAS FORMULATIONS.

**Claims**

1. A solution composition capable of providing synergistic broad-spectrum antimicrobial activity, consisting essentially of an aqueous solvent, sodium salt(s) of EDTA, D-mannitol and ethanol; wherein the sodium salt(s) of EDTA is at a concentration ranging between 0.06 to 8% (w/v), D-mannitol is at a concentration ranging between 0.5 to 10% (w/v), and ethanol is at a concentration ranging between 6.25 to 25% (v/v), with a pH ranging between 7 to 11.

2. The composition according to claim 1, wherein pH of the composition is 7.5+/-0.5.

3. The composition according to claim 1, wherein D-mannitol is at a concentration of 0.73% (w/v).

4. The composition according to claim 1 or 2, wherein the sodium salt(s) of EDTA is at a concentration of 1 to 4% (w/v), D-mannitol is at a concentration of 0.5 to 1% (w/v) and ethanol is at a concentration of 10 to 25% (v/v).

5. The composition according to any one of claims 1 to 4, for use as a catheter lock-flush solution.

6. The composition according to any one of claims 1 to 4, for use as disinfectant and/or maintaining catheter patency.

7. A solution composition according to any one of claims 1 to 4 for use in a method for disinfecting a catheter comprising: introducing the solution into an interior lumen of the catheter.

8. The method for disinfecting a catheter according to claim 7, wherein pH of the solution is 7.5+/-0.5.

9. A solution composition according to any one of claims 1 to 4 for use in a method for maintaining catheter patency, wherein said method comprises introducing the solution into an interior lumen of the catheter.

10. The method according to claim 9, wherein pH of the solution is 7.5+/-0.5.

**Patentansprüche**

1. Lösungszusammensetzung, die eine synergistische antimikrobielle Breitbandaktivität bereitstellen kann, im Wesentlichen aus einem wässrigen Lösungsmittel, Natriumsalz(en) von EDTA, D-Mannitol und Ethanol besteht; wobei das/die Natriumsalz(e) von EDTA in einer Konzentration im Bereich zwischen 0,06 bis 8 % (m/V) vorliegt/vorliegen, D-Mannitol in einer Konzentration im Bereich zwischen 0,5 bis 10 % (m/V) vorliegt und Ethanol in einer Konzentration im Bereich zwischen 6,25 bis 25 % (V/V) vorliegt, mit einem pH im Bereich zwischen 7 bis 11.

**2.** Zusammensetzung nach Anspruch 1, wobei der pH der Zusammensetzung 7,5 ± 0,5 beträgt.

**3.** Zusammensetzung nach Anspruch 1, wobei D-Mannitol in einer Konzentration von 0,73 % (m/V) vorliegt.

**4.** Zusammensetzung nach Anspruch 1 oder 2, wobei das/die Natriumsalz(e) von EDTA in einer Konzentration von 1 bis 4 % (m/V) vorliegt/vorliegen, D-Mannitol in einer Konzentration von 0,5 bis 1 % (m/V) vorliegt und Ethanol in einer Konzentration von 10 bis 25 % (V/V) vorliegt.

**5.** Zusammensetzung nach einem der Ansprüche 1 bis 4 für die Verwendung als eine Katheter-Lock-Spüllösung.

**6.** Zusammensetzung nach einem der Ansprüche 1 bis 4 für die Verwendung als Desinfektionsmittel und/oder zur Erhaltung von Katheterdurchgängigkeit.

**7.** Lösungszusammensetzung nach einem der Ansprüche 1 bis 4 für die Verwendung in einem Verfahren zur Desinfizierung eines Katheters, das die Einführung der Lösung in ein inneres Lumen des Katheters umfasst.

**8.** Verfahren zur Desinfizierung eines Katheters nach Anspruch 7, wobei der pH der Lösung 7,5 ± 0,5 beträgt.

**9.** Lösungszusammensetzung nach einem der Ansprüche 1 bis 4 für die Verwendung in einem Verfahren zur Erhaltung von Katheterdurchgängigkeit, wobei das Verfahren die Einführung der Lösung in ein inneres Lumen des Katheters umfasst.

**10.** Verfahren nach Anspruch 9, wobei der pH der Lösung 7,5 ± 0,5 beträgt.

## Revendications

**1.** Composition en solution, capable de fournir une activité antimicrobienne à large spectre synergique, constituée essentiellement d'un solvant aqueux, de sel(s) sodique(s) d'EDTA, de D-mannitol et d'éthanol ; dans laquelle le ou les sels sodiques d'EDTA se trouvent selon une concentration dans la plage allant de 0,06 à 8% (p/v), le D-mannitol se trouve selon une concentration dans la plage allant de 0,5 à 10% (p/v), et l'éthanol se trouve selon une concentration dans la plage allant de 6,25 à 25% (v/v), avec un pH dans la plage allant de 7 à 11.

**2.** Composition selon la revendication 1, dans laquelle le pH de la composition est de 7,5 ± 0,5.

**3.** Composition selon la revendication 1, dans laquelle le D-mannitol se trouve selon une concentration de 0,73% (p/v).

**4.** Composition selon la revendication 1 ou 2, dans laquelle le ou les sels sodiques d'EDTA se trouvent selon une concentration allant de 1 à 4% (p/v), le D-mannitol se trouve selon une concentration allant de 0,5 à 1% (p/v), et l'éthanol se trouve selon une concentration allant de 10 à 25% (v/v).

**5.** Composition selon l'une quelconque des revendications 1 à 4, pour une utilisation comme solution de rinçage-verrou de cathéter.

**6.** Composition selon l'une quelconque des revendications 1 à 4, pour une utilisation comme désinfectant et/ou pour le maintien de la perméabilité d'un cathéter.

**7.** Composition en solution selon l'une quelconque des revendications 1 à 4, pour une utilisation dans une méthode de désinfection d'un cathéter, comprenant :
l'introduction de la solution dans une lumière intérieure du cathéter.

**8.** Méthode de désinfection d'un cathéter selon la revendication 7, dans laquelle le pH de la solution est de 7,5 ± 0,5.

**9.** Composition en solution selon l'une quelconque des revendications 1 à 4, pour une utilisation dans une méthode de maintien de la perméabilité d'un cathéter, ladite méthode comprenant l'introduction de la solution dans une lumière intérieure du cathéter.

**10.** Méthode selon la revendication 9, dans laquelle le pH de la solution est de 7,5 ± 0,5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6350251 B
- EP 1644024 A2
- EP 1628655 A4
- WO 2015048559 A1
- US 20090170947 A1

**Non-patent literature cited in the description**

- **DAOUICHER et al.** *New England Journal Of Medicine,* 1999, vol. 340, 1-8
- Complications Associated With Peripherally Inserted Central Catheter Use During Pregnancy. *AM. J. OBSTET. GYCOL.,* May 2003, vol. 188 (5), 1223-5
- *Clinical Nutrition,* 2002, vol. 21 (1), 33-38
- *Science News,* 14 July 2001, 1-5
- **JUSTO JA ; BOOKSTAVER PB.** *Infect Drug Resist,* 12 December 2014, vol. 7, 343-63
- *Kidney International,* September 2002
- **PIERCE DA ; ROCCO MV.** *Pharmacotherapy,* November 2010, vol. 30 (11), 1150-8
- **JOHN E. MORAN ; STEPHEN R. ASH.** *Locking Solutions for Hemodialysis Catheters,* September 2008, vol. 21 (5), 490-2
- **BARRAUD N ; BUSON A ; JAROLIMEK W ; RICE SA.** Mannitol Enhances Antibiotic Sensitivity of Persister Bacteria in Pseudomonas aeruginosa Bio-films. *PLoS ONE,* 2013, vol. 8 (12), e84220
- **CMICH C. J. ; DUSTER M. ; JONES A. ; MAKI D. G.** Prospective Randomized Double-Blind Trial of an Ethanol Lock for Prevention of CLABSI Proceedings 49. *Interscience Conference on Antimicrobial Agents and Chemotherapy, San Francisco, CA,* 12 September 2009
- **ISSAM RAAD et al.** *Antimicrob. Agents Chemother,* January 2007, vol. 51 (1), 78-83
- **BEATRIZ PASSERINI DE ROSSI et al.** *Journal of Medical Microbiology,* 2012, vol. 61, 1248-1253
- **M.L MACKAY ; K MILINE ; I. M. GOULD.** Comparison of methods for assessing synergic antibiotic interactions. *International journal of antimicrobial agents,* 2000, vol. 15, 125-129